# FASCICULE DE BREVET EUROPEEN

(11) **EP 0 821 924 B1**
(45) Date de publication et mention de la délivrance du brevet: **10.09.2003**
(21) Numéro de dépôt: 97420135.2
(22) Date de dépôt: 28.07.1997
(51) Int. Cl.: A61F 2/40

(54) **Prothèse ajourée de l'extrémité supérieure de l'humérus**
Durchlochte Humeruskopfprothese
Through-hole prosthesis for the upper humerus

(30) Priorité: 02.08.1996 FR 9609976
(43) Date de publication de la demande: 04.02.1998
(73) Titulaire: TORNIER SA, 38330 Saint-Ismier (FR)
(72) Inventeur: Boileau, Pascal, 06300 Nice (FR); Walch, Gilles, 69003 Lyon (FR)
(74) Mandataire: Myon, Gérard Jean-Pierre

(56) Documents cités:
- FR-A- 2 689 758
- GB-A- 1 340 451
- US-A- 3 228 393
- US-A- 4 919 670
- US-A- 5 019 108

## Description

La présente invention a trait à une prothèse humérale comprenant une queue destinée à être ancrée dans le canal huméral d'un patient, une partie métaphysaire prolongeant ladite queue vers le haut et vers l'intérieur, ladite partie métaphysaire se raccordant, au niveau d'une zone de raccord, à une collerette servant de support à une calotte sensiblement hémisphérique apte à coopérer avec la glène de l'épaule, ladite zone de raccord étant située sensiblement selon un axe médian de la collerette.

Lors de la fracture de l'extrémité haute d'un humérus, ce dernier se brise généralement en plusieurs morceaux, en particulier la diaphyse, les tubérosités humérales et enfin la tête de l'humérus qui coopère avec la glène de l'épaule.

Lors de la réduction d'une telle fracture, seule la tête humérale doit être remplacée par une calotte sensiblement hémisphérique, alors que les autres morceaux fracturés peuvent être reconstruits autour d'une prothèse.

De manière connue, une telle prothèse comprend généralement une queue, ou partie diaphysaire, introduite dans le canal huméral de la diaphyse de l'os. Cette queue est prolongée par une partie métaphysaire recourbée vers le haut et vers l'intérieur, en faisant référence à une prothèse portée par un patient debout, autour de laquelle sont rassemblés les tubérosités constituant la métaphyse de l'os en vue de leur reconstruction. La partie métaphysaire de la prothèse se raccorde à une collerette de la réception de la calotte remplaçant la tête humérale.

Ce type de prothèses, telles que par exemple celles dites de NEER, présentent toutefois certains inconvénients. En effet, elles sont réalisées en un seul bloc de sorte qu'il faut posséder un grand nombre d'implants pour répondre aux exigences anatomiques des divers patients. De plus, ces prothèses présentent une seule taille de queue et de calotte, et cette dernière ne peut pas être déportée comme cela s'avère nécessaire dans certains cas. En outre, le volume de la partie supérieure de cette prothèse ne permet pas, lors de fractures importantes de la partie supérieure de l'humérus, une reconstruction satisfaisante des fragments osseux entre eux.

On connaît en outre, par le FR-A-2 689 758, une prothèse humérale comprenant, dans sa partie métaphysaire, une ailette de réinsertion. Cette dernière présente des perforations permettant le passage de fils qui assurent la fixation des os à la prothèse. Toutefois, ces perforations, qui sont de très faible diamètre, ne permettent pas non plus un rapprochement des fragments osseux entre eux. De la sorte, la consolidation mutuelle de ces fragments se révèle insuffisante, au détriment de l'intégrité physique du patient.

L'invention se propose donc de réaliser une prothèse humérale du type précité, qui permette de pallier les inconvénients de l'art antérieur évoqués ci-dessus.

A cet effet, l'invention a pour objet une prothèse humérale du type précité, caractérisée en que la zone de raccord s'étend uniquement sur une fraction de l'axe médian de manière à définir, à l'extérieur de la zone de raccord ou à l'extérieur d'une portion de la zone de raccord, un volume libre de rapprochement et de fusion des fragments d'os de la métaphyse.

L'invention permet de réaliser les objectifs précédemment mentionnés. En effet, la présence d'un volume libre permet la formation d'un amas de fragments, voire la mise en place d'un greffon afin de favoriser l'osteosynthèse postérieure à l'intervention chirurgicale. La prothèse conforme à l'invention présente ainsi, par rapport aux prothèses de l'art antérieur pour lesquelles les fragments osseux de la métaphyse étaient uniquement fixés à la prothèse, l'avantage que ces fragments osseux peuvent être rapprochés et fixés entre eux sensiblement selon leur encombrement et leur configuration originels. Ceci permet une consolidation osseuse nettement meilleure.

Le volume de rapprochement et de fusion des fragments d'os est situé à la partie externe du volume général de la partie métaphysaire. En effet, c'est cette partie proximale externe qui apparaît la plus difficile à consolider, en raison des transferts de contraintes que cette portion osseuse doit supporter lors du fonctionnement normal d'une épaule.

Selon une caractéristique avantageuse de l'invention, l'axe médian de la collerette est un diamètre sensiblement coplanaire avec l'axe de la queue.

Selon une caractéristique supplémentaire de l'invention, la partie métaphysaire comprend une unique branche prolongeant la queue humérale et se raccordant à la collerette au niveau d'une zone de raccord de manière à former, à l'extérieur de la zone de raccord, le volume libre de rapprochement et de fusion des fragments d'os de la métaphyse. Ce mode de réalisation permet de délimiter un vaste espace libre en vue du rapprochement et de la consolidation des fragments osseux entre eux.

Cette unique branche peut présenter une concavité dirigée vers l'intérieur. Elle peut être pourvue d'au moins un aileron aminci par rapport à cette branche, dirigé vers l'intérieur et/ou l'extérieur, et dans lequel sont ménagées des perforations. Ces perforations sont destinées au passage de fils de suture permettant de fixer les fragments osseux par rapport à la prothèse, en vue d'une meilleure reconstitution. Le fait que l'aileron soit aminci par rapport à la branche unique assure une meilleure mise en place des différents fragments osseux les uns par rapport aux autres, et garantit un contact intime entre ces derniers.

Selon une caractéristique supplémentaire de l'invention, la partie métaphysaire comprend deux branches intérieure et extérieure s'étendant à partir de la queue et se raccordant à la collerette au niveau de zones de raccord de manière à définir, entre ces zones de raccord, un évidement qui forme ledit volume libre de rapprochement et de fusion des fragments d'os de la métaphyse. Cette branche extérieure constitue une surface d'appui pour l'os spongieux, et évite à ce dernier de s'affaisser.

Dans ce cas, la branche extérieure peut être pourvue d'un aileron aminci par rapport à celle-ci, dirigé vers l'extérieur et dans lequel sont ménagées des perforations destinées au passage de fils de suture.

Selon une caractéristique préférée de l'invention, la partie métaphysaire comprend une branche prolongeant la queue humérale et se raccordant à la collerette au niveau d'une zone de raccord, ainsi qu'un ergot faisant saillie à partir de la collerette en direction opposée à l'emplacement de la calotte, et à l'extérieur de la branche, le volume libre de rapprochement et de fusion des fragments d'os de la métaphyse étant défini entre la zone de raccord de la branche et la zone de raccord de l'ergot. La présence de l'ergot évite à l'os spongieux de s'affaisser du fait de l'appui procuré par cet ergot. De plus, étant donné que ce dernier ne s'étend pas jusqu'à la queue humérale, le volume de rapprochement et de fusion des fragments d'os de la métaphyse, défini entre la branche extérieure, l'ergot et la collerette, est ouvert au niveau de la queue. Ce volume libre possède donc des dimensions importantes et permet une consolidation satisfaisante des fragments osseux entre eux.

L'ergot peut être pourvu d'un aileron aminci par rapport à cet ergot, dirigé vers l'extérieur et dans lequel sont ménagées des perforations destinées au passage de fils de suture.

L'invention va être décrite ci-dessous, en référence aux dessins annexés donnés uniquement à titre d'exemple non limitatif, et dans lesquels :
- la figure 1 est une vue en perspective éclatée illustrant un premier mode de réalisation d'une prothèse humérale conforme à l'invention ;
- la figure 2 est une coupe suivant la ligne II-II à la figure 1 ;
- les figures 3 et 4 sont des vues en perpsective analogues à la figure 1, illustrant des second et troisième modes de réalisation de l'invention.

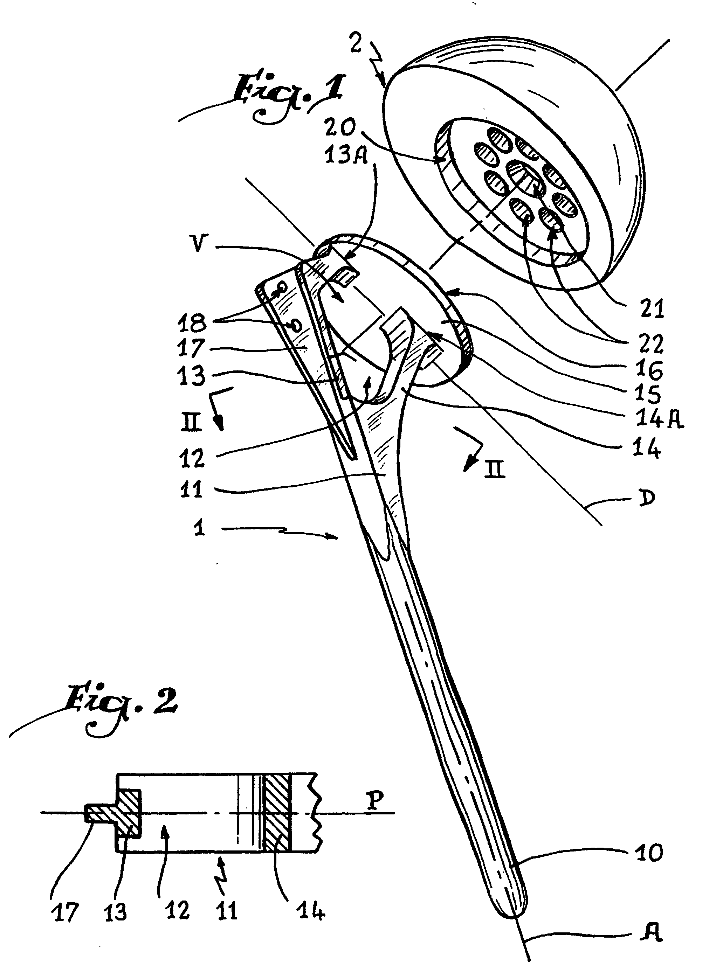

La prothèse représentée à la figure 1 et désignée dans son ensemble par la référence 1, est destinée à supporter une calotte hémisphèrique 2 apte à coopérer avec la glène de l'épaule d'un patient.

Cette prothèse comprend une queue 10 de section sensiblement circulaire qui s'engage dans le canal huméral, prolongée par une partie métaphysaire 11 à laquelle est raccordée une collerette 15.

Cette partie métaphysaire comprend une branche extérieure 13 et une branche intérieure 14, qui se raccordent chacune à la collerette par des zones de raccord respectives 13A, 14A. Ces dernières sont disposées sensiblement sur un axe médian de la collerette, à savoir un diamètre (D) disposé dans le même plan (P) que l'axe (A) de la queue 10 de la prothèse. Ces zones de raccord, 13A, 14A s'étendent uniquement sur une fraction de ce diamètre de manière à définir, entre les branches 13 et 14, un évidement 12 qui forme un volume libre (V) de rapprochement et de fusion des fragments d'os de la métaphyse, au sein duquel les fragments osseux éclatés peuvent être rapprochés en vue de leur consolidation.

De plus, on peut prévoir que l'évidement 12 soit rempli, avant introduction de la queue 10 dans le canal huméral, de fragments osseux ou substitut découpés au profil de l'évidement.

La collerette 15 présente une face inclinée 16 qui coopère avec un emboîtement 20 circulaire ménagé dans la collerette, de manière excentrée par rapport à l'axe de cette dernière, le diamètre de cet emboîtement correspondant, au jeu près, à celui de ladite collerette. Au centre de cet emboîtement est ménagé un alésage tronconique 21 dont le profil correspond à celui d'un pion non représenté solidaire de la collerette 15. Une série de trous 22 est réalisée au fond de l'emboîtement 20, à la périphérie externe de l'alésage 21.

Comme le montre en particulier la figure 2, la branche extérieure 13 présente une section inférieure à celle de la branche 14, afin d'assurer un meilleur rapprochement des fragments osseux entre eux lors de l'introduction de la queue 10 dans le canal huméral. Cette branche extérieure 13 confère en outre un appui à l'os spongieux de manière à en empêcher l'affaissement.

La branche extérieure 13 se prolonge vers l'extérieur par un aileron 17 dont la dimension transversale est inférieure à celle de la branche 13. Cet aileron comprend des perforations 18 permettant le passage de fils de suture.

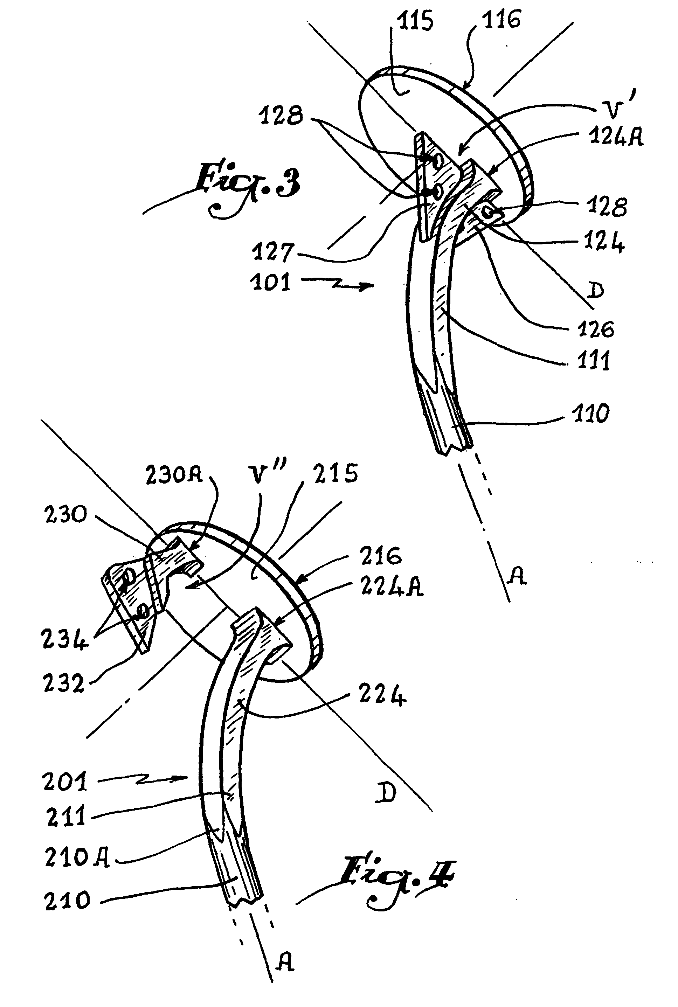

La figure 3 montre un second mode de réalisation de la prothèse conforme à l'invention. Sur cette figure, les éléments analogues à ceux représentés aux figures 1 et 2 portent les mêmes numéros de référence augmentés de 100.

La prothèse 101 diffère de la prothèse 1 représentée à la figure 1 par le fait que sa partie métaphysaire 111 n'est plus constituée par deux branches, mais par une unique branche 124 dont la concavité est dirigée vers l'intérieur. Cette branche unique 124 prolonge la queue 110 de manière à se raccorder à la collerette 115 au niveau d'une zone de raccordement 124A disposée sensiblement sur le diamètre (D) de la collerette, coplanaire avec l'axe (A) de la queue 110 de la prothèse. Cette zone de raccord 124A s'étend uniquement sur une fraction de ce diamètre de manière à définir, à l'extérieur de la branche 124, un volume libre (V') de rapprochement et de fusion des fragments d'os de la métaphyse.

Deux ailerons respectivement intérieur 126 et extérieur 127 font saillie à partir de la face de la collerette opposée à la face 116 de réception de la calotte 102. Chacun de ces ailerons 126, 127 s'étend jusqu'aux faces respectivement extérieure et intérieure de la branche unique 124. Les dimensions transversales de ces ailerons sont nettement inférieures à celles de la branche 124, de manière à permettre un rapprochement plus aisé des fragments osseux entre eux. Chacun de ces ailerons est pourvu de perforations 128 destinées au passage de fils de suture. Bien entendu, il serait possible de prévoir au voisinage de la branche 124, uniquement l'aileron extérieur 127, ou bien encore uniquement l'aileron intérieur 126.

La figure 4 représente un troisième mode de réalisation de la prothèse conforme à l'invention. Dans ce troisième mode, les éléments analogues à ceux décrits aux figures 1 et 2 seront affectés des mêmes numéros de référence augmentés de 200, alors que les éléments analogues à ceux représentés à la figure 3 seront affectés des mêmes numéros de référence augmentés de 100.

La prothèse 201 représentée à la figure 4 diffère de celle représentée à la figure 3 en ce que la branche 224 est dépourvue d'ailerons. La partie métaphysaire 211 de la prothèse 201 est complétée par un ergot 230 faisant saillie à partir de la portion externe de la collerette, en direction opposée à la calotte 202. La branche 224 et l'ergot 230 se raccordent chacun à la collerette 215 par des zones de raccord respectives 224A et 230A. Ces dernières sont disposées sensiblement sur le diamètre (D) de la collerette, coplanaire avec l'axe (A) de la queue 210 de la prothèse. Ces zones de raccord s'étendent uniquement sur une fraction de ce diamètre de manière à définir, entre la branche 224 et l'ergot 230, un volume libre (V'') de rapprochement et de fusion des fragments d'os de la métaphyse.

L'ergot 230 est prolongé par un aileron 232 s'évasant en direction de la queue 210. Cet aileron 232 présente des dimensions transversales inférieures à celles de l'ergot 230, et se trouve pourvu de perforations 234 permettant le passage de fils de suture.

Etant donné que l'ergot ne s'étend pas jusqu'à la partie proximale 210A de la queue 210, le volume libre (V'') de rapprochement et de fusion des fragments d'os entre eux est ouvert au niveau de cette partie 210A. De plus, la présence de l'ergot 230 assure un appui à l'os spongieux et en empêche l'affaissement.

## Revendications

1. Prothèse humérale (1 ; 101 ; 201) comprenant une queue (10 ; 110 ; 210) destinée à être ancrée dans le canal huméral d'un patient, une partie métaphysaire (11 ; 111 ; 211) prolongeant ladite queue vers le haut et vers l'intérieur, ladite partie métaphysaire se raccordant, au niveau d'une zone de raccord, (13A, 14A ; 124A ; 224A, 230A) à une collerette (15 ; 115 ; 215) servant de support à une calotte (2 ; ; 102 ; 202) sensiblement hémisphérique apte à coopérer avec la glène de l'épaule, ladite zone de raccord étant située sensiblement selon un axe médian (D) de ladite collerette, **caractérisée en ce que** ladite zone de raccord s'étend uniquement sur une fraction dudit axe médian (D) de manière à définir, à l'extérieur de ladite zone de raccord (124A) ou à l'extérieur d'une portion (14A ; 224A) de ladite zone de raccord (13A, 14A ; 224A, 230A), un volume libre (V ; V' ; V'') de rapprochement et de fusion des fragments d'os de la métaphyse.

2. Prothèse selon la revendication 1, **caractérisée en ce que** ledit axe médian de la collerette (15 ; 115 ; 215) est un diamètre (D) sensiblement coplanaire avec l'axe (A) de la queue (10, 110, 210).

3. Prothèse selon la revendication 1 ou 2, **caractérisée en ce que** la partie métaphysaire (111) comprend une unique branche (124) prolongeant la queue humérale (110) et se raccordant à ladite collerette (115) au niveau d'une zone de raccord (124A) de manière à former, à l'extérieur de ladite zone de raccord (124A), ledit volume libre (V') de rapprochement et de fusion des fragments d'os de la métaphyse.

4. Prothèse selon la revendication 3, **caractérisée en ce que** ladite unique branche (124) présente une concavité dirigée vers l'intérieur.

5. Prothèse selon la revendication 3 ou 4, **caractérisée en ce que** ladite unique branche (124) est pourvue d'au moins un aileron (126, 127) aminci par rapport à ladite branche, dirigé vers l'intérieur et/ou l'extérieur et dans lequel sont ménagées des perforations (128).

6. Prothèse selon la revendication 1 ou 2, caractérisée en que la partie métaphysaire (11) comprend deux branches intérieure (14) et extérieure (13) s'étendant à partir de la queue (10) et se raccordant à la collerette au niveau de zones de raccord (14A, 13A), de manière à définir, entre lesdites zones de raccord (14A, 13A) un évidement (12) qui forme ledit volume libre (V) de rapprochement et de fusion des fragments d'os de la métaphyse.

7. Prothèse selon la revendication 6, **caractérisée en ce que** la branche extérieure (13) est pourvue d'un aileron (17) aminci par rapport à ladite branche, et dans lequel sont ménagées des perforations (18).

8. Prothèse selon la revendication 1 ou 2, **caractérisée en ce que** la partie métaphysaire (211) comprend une branche (224) prolongeant la queue humérale (210) et se raccordant à ladite collerette (215) au niveau d'une zone de raccord (224A), ainsi qu'un ergot (230) faisant saillie à partir de la collerette (215) en direction opposée à l'emplacement de la calotte (202), et à l'extérieur de ladite branche (224), ledit volume libre (V'') de rapprochement et de fusion des fragments d'os de la métaphyse étant défini entre la zone de raccord (224A) de la branche (224) et la zone de raccord (230A) de l'ergot (230).

9. Prothèse selon la revendication 8, **caractérisée** en que ledit ergot (230) est pourvu d'un aileron (232) aminci par rapport audit ergot, dirigé vers l'extérieur et dans lequel sont ménagées des perforations (234).

## Patentansprüche

1. Oberarmprothese (1; 101; 201), umfassend ein Endstück (10; 110; 210), das dazu bestimmt ist, in der Oberarmröhre eines Patienten verankert zu werden, wobei ein metaphysärer Teil (11; 111; 211) das Endstück nach oben und nach innen verlängert, und der metaphysäre Teil sich auf Höhe einer Anschlusszone (13A, 14A; 124A; 224A, 230A) an einen Kragen (15; 115; 215) anschließt, der als Stütze für eine im Wesentlichen halbkugelförmige Kappe (2; 102; 202) dient, die mit der Schultergelenkpfanne zusammenwirken kann, wobei die Anschlusszone im Wesentlichen entlang einer Mittelachse (D) des Kragens angeordnet ist, **dadurch gekennzeichnet, dass** sich die Anschlusszone nur auf einem Bruchteil der Mittelachse (D) erstreckt, um außerhalb der Anschlusszone (124A) oder außerhalb eines Abschnittes (14A; 224A) der Anschlusszone (13A, 14A; 224A, 230A) ein freies Volumen (V; V'; V'') für die Annäherung und Verschmelzung der Knochenfragmente der Metaphysis zu definieren.

2. Prothese nach Anspruch 1, **dadurch gekennzeichnet, dass** die Mittelachse des Kragens (15; 115; 215) ein im Wesentlichen mit der Achse (A) des Endstücks (10, 110, 210) koplanarer Durchmesser (D) ist.

3. Prothese nach Anspruch 1 oder 2, **dadurch gekennzeichnet, dass** der metaphysäre Teil (111) einen einzigen Schenkel (124) umfasst, der das Oberarmendstück (110) verlängert und sich an den Kragen (115) auf Höhe einer Anschlusszone (124A) anschließt, um außerhalb der Anschlusszone (124A) das freie Volumen (V') für die Annäherung und Verschmelzung der Knochenfragmente der Metaphysis zu bilden.

4. Prothese nach Anspruch 3, **dadurch gekennzeichnet, dass** der einzige Schenkel (124) eine nach innen gerichtete Konkavität aufweist.

5. Prothese nach Anspruch 3 oder 4, **dadurch gekennzeichnet, dass** der einzige Schenkel (124) mit mindestens einem Flügel (126, 127) versehen ist, der in Bezug auf den Schenkel dünner ist und nach innen und/oder außen gerichtet ist, und in dem Löcher (128) vorgesehen sind.

6. Prothese nach Anspruch 1 oder 2, **dadurch gekennzeichnet, dass** der metaphysäre Teil (11) zwei Schenkel, einen inneren (14) und einen äußeren (13) umfasst, die sich ausgehend von dem Endstück (10) erstrecken und sich an den Kragen auf Höhe von Anschlusszonen (14A, 13A) anschließen, um zwischen den Anschlusszonen (14A, 13A) eine Aussparung (12) zu definieren, die das freie Volumen (V) für die Annäherung und Verschmelzung der Knochenfragmente der Metaphysis bildet.

7. Prothese nach Anspruch 6, **dadurch gekennzeichnet, dass** der äußere Schenkel (13) mit einem Flügel (17) versehen ist, der in Bezug auf den Schenkel dünner ist und in dem Löcher (18) vorgesehen sind.

8. Prothese nach Anspruch 1 oder 2, **dadurch gekennzeichnet, dass** der metaphysäre Teil (211) einen Schenkel (224), der das Oberarmendstück (210) verlängert und sich an den Kragen (215) auf Höhe der Anschlusszone (224A) anschließt, sowie einen Haken (230) umfasst, der von dem Kragen (215) in die zur Anordnung der Kappe (202) entgegengesetzte Richtung und außerhalb des Schenkels (224) vorspringt, wobei das freie Volumen (V'') für die Annäherung und Verschmelzung der Knochenfragmente der Metaphysis zwischen der Anschlusszone (224A) des Schenkels (224) und der Anschlusszone (230A) des Hakens (230) definiert ist.

9. Prothese nach Anspruch 8, **dadurch gekennzeichnet, dass** der Haken (230) mit einem Flügel (232) versehen ist, der in Bezug auf den Haken dünner ist und nach außen gerichtet ist und in dem Löcher (234) vorgesehen sind.

## Claims

1. Humeral prosthesis (1; 101; 201) comprising a tail (10; 110; 210) intended to be anchored in the humeral canal of a patient, a metaphysiary part (11; 111; 211) extending the said tail upwards and inwards, the said metaphysiary part joining, at a connection zone (13A, 14A; 124A; 224A, 230A), onto a flange (15; 115; 215) serving as support for an essentially hemispherical cap (2; 102; 202) able to interact with the glenoid cavity of the shoulder, the said connection zone being located essentially along a median axis (A) of the flange, **characterised in that** the said connection zone extends only over a fraction of the said median axis (D) so as to define, on the outside of the said connection zone (124A) or on the outside of a portion (14A; 224A) of the said connection zone (13A, 14A; 224A, 230A), a free volume (V; V'; V") for drawing together and fusion of the bone fragments of the metaphysis.

2. Prosthesis according to Claim 1, **characterised in that** the said median axis of the flange (15; 115; 215) is a diameter (D) essentially coplanar with the axis (A) of the tail (10, 110, 210).

3. Prosthesis according to Claim 1 or 2, **characterised in that** the metaphysiary part (111) comprises a single branch (124) extending the humeral tail (110) and joining onto the said flange (115) at a connection zone (124A) so as to form, on the outside of the said connection zone (124A), the said free volume (V') for drawing together and fusion of the bone fragments of the metaphysis.

4. Prosthesis according to Claim 3, **characterised in that** the said single branch (124) has a concavity facing inwards.

5. Prosthesis according to Claim 3 or 4, **characterised in that** the said single branch (124) is provided with at least one fin (126, 127) that is made thinner with respect to said branch and is oriented inwards and/or outwards and in which perforations (128) have been made.

6. Prosthesis according to Claim 1 or 2, **characterised in that** the metaphysiary part (11) comprises two branches, internal (14) and external (13), extending from the tail (10) and joining onto the flange at the said connection zones (14A, 13A) so as to define, between the said connection zones (14A, 13A), a cavity (12) which forms the said free volume (V) for drawing together and fusion of the bone fragments of the metaphysis.

7. Prosthesis according to Claim 6, **characterised in that** the external branch (13) is provided with a fin (17) that is made thinner with respect to the said branch and in which perforations (18) have been made.

8. Prosthesis according to Claim 1 or 2, **characterised in that** the metaphysiary part (211) comprises a branch (224) extending the humeral tail (210) and joining onto the said flange (215) at a connection zone (224A), as well as a lug (230) protruding from the flange (215) in the direction opposed to the location of the cap (202), and on the outside of the said branch (224) the said free volume (V") for drawing together and fusion of the bone fragments of the metaphysis being defined between the connection zone (224A) of the branch (224) and the connection zone (230A) of the lug (230).

9. Prosthesis according to Claim 8 **characterised in that** the said lug (230) is provided with a fin (232) that is made thinner with respect to this lug and is oriented outwards and in which perforations (234) have been made.
